(19) Europäisches Patentamt
European Patent Office
Office européen des brevets

(11) Veröffentlichungsnummer: **0 202 349 B1**

(12) **EUROPÄISCHE PATENTSCHRIFT**

(45) Veröffentlichungstag der Patentschrift: **27.02.91**

(51) Int. Cl.5: **C07D 207/08**, A61K 31/40

(21) Anmeldenummer: **85106219.0**

(22) Anmeldetag: **21.05.85**

Die Akte enthält technische Angaben, die nach dem Eingang der Anmeldung eingereicht wurden und die nicht in dieser Patentschrift enthalten sind.

# (54) Neue Pyrrolidiniumsulfobetaine mit Carbonamidgruppen und Verfahren zu ihrer Herstellung.

(43) Veröffentlichungstag der Anmeldung:
**26.11.86 Patentblatt 86/48**

(45) Bekanntmachung des Hinweises auf die Patenterteilung:
**27.02.91 Patentblatt 91/09**

(84) Benannte Vertragsstaaten:
**AT BE CH DE FR GB IT LI NL SE**

(56) Entgegenhaltungen:
**EP-A- 0 095 241**
**DD-A- 154 444**
**GB-A- 2 080 293**

**W.M. LINFIELD, JOURNAL OF THE AMERICAN OIL CHEMISTS' SOCIETY, Vol. 55 (1978) 87, 741**

(73) Patentinhaber: **Akademie der Wissenschaften der DDR**
**Otto-Nuschke-Strasse 22/23**
**DDR-1086 Berlin(DD)**

(72) Erfinder: **Ballschuh, Detlef, Dr. Dipl.-Chem.**
**Graudenzer Strasse 17**
**DDR-1034 Berlin(DD)**
Erfinder: **Ohme, Roland, Dr. Dipl.-Chem.**
**Waldstrasse 6**
**DDR-1180 Berlin(DD)**
Erfinder: **Rusche, Jochen, Dr. Dipl.-Chem.**
**Hans-Loch-Strasse 263**
**DDR-1136 Berlin(DD)**
Erfinder: **Seibt, Horst, Dr. Dipl.-Chem.**
**Eugen-Schönhaar-Strasse 15**
**DDR-1055 Berlin(DD)**

(74) Vertreter: **Patentanwälte Beetz sen. - Beetz jun. Timpe - Siegfried - Schmitt-Fumian**
**Steinsdorfstrasse 10**
**D-8000 München 22(DE)**

EP 0 202 349 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Pyrrolidiniumsulfobetaine, die als zusätzliches polares Strukturelement die Carbonamidgruppe enthalten und die allgemeine Formel 1 besitzen,

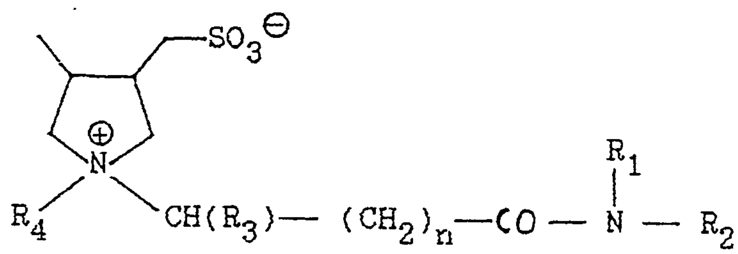

(I),

worin bedeuten:

- $R_1$ Waserstoff, geradkettiges oder verzweigtes Alkyl mit bis zu 22 C-Atomen,
- $R_2$ Wasserstoff, $-NH_2$ oder kurzkettiges Alkyl mit bis zu 4 C-Atomen,
- $R_3$ Wasserstoff, Alkylaminocarbonyl oder Alkyl mit bis zu 22 C-Atomen,
- $R_4$ Wasserstoff oder Alkyl oder Hydroxyalkyl mit bis zu 22 C-Atomen

und

n 0, 1, 2 oder 3,

Verfahren zu ihrer Herstellung sowie ihre Verwendung. Sulfobetaine der allgemeinen Formel 1 erhöhen die Leitfähigkeit und können deshalb als leitfähige Beschichtungsmittel eingestezt werden. Wenn einer der Substituenten $R_1$, $R_3$ oder $R_4$ langkettig ist (8 bis 22 C-Atome), so haben diese Pyrrolidiniumsulfobetaine wertvolle Tensideigenschaften. Es ist bekannt, daß zusätzliche Carbonamidgruppen die Tensideigenschaften von Sulfobetainen verbessern. Nach Lin field et al., J. Amer. Oil Chem. Soc. 55 (1978) 87; 741, haben solche Tenside von den bisher bekannten das beste Kalkseifendispergiervermögen. Die bisher bekannten Vertreter dieser Verbindungsklasse enthalten die Carbonamidgruppe in der Reihenfolge der Atomgruppen -CO- und -NH-. Sie sind Derivate von 1,2- und 1,3- Diaminen und haben die allgemeine Formel II, in der $R_5$ Alkylreste, $R_6$ Methyl- oder Hydroxyalkylgruppen und n die Zahlen 2 oder 3 bedeuten.

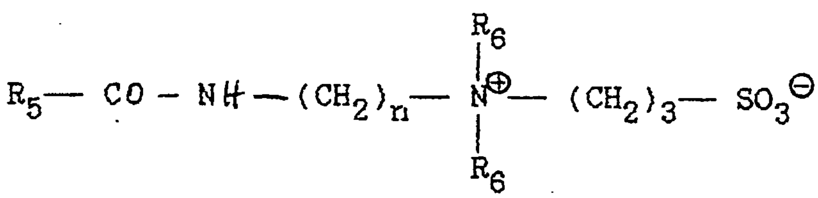

(II).

Das Verfahren zur Herstellung dieses Verbindungstyps hat für den technischen Einsatz erhebliche Nachteile: Es benötigt unsymmetrische dialkylierte 1,2- oder 1,3-Diamine als Ausgangsstoffe und verwendet das hochgradig cancerogene Propansulton zum Aufbau der Sulfobetainstruktur. Aus GB-A-2 080 293 sind ferner 3-Methyl-4-sulfomethylpyrrolidiniumbetaine bekannt, die am Ammoniumstickstoffatom mit $C_{1-22}$-Alkyl und/oder Benzyl substituiert sind. Diese Verbindungen werden durch Sulfocyclisierung von entsprechenden Diallylammoniumsalzen mit molaren Mengen gepufferten Hydrogensulfits ($HSO_3^-/SO_3^{--}$) hergestellt, die durch Sauerstoff und Schwermetallionen induziert ist und radikalisch verläuft. Formal erfolgt dabei eine homogenkatalytische 1,7-Addition des Hydrogensulfits an das Diallylammoniumsalz. Die Sulfoyclisierungsreaktion verläuft optimal im Temperaturbereich von 10 bis 80 °C in wäßrigem Medium. Verbindungen dieser Substanzklasse, die noch zusätzliche funktionelle Gruppen in einer N-Alkylkette tragen, sind bisher nicht bekannt geworden. Der Erfindung liegt die Aufgabe zugrunde, neuartige 3-Sulfomethylpyrrolidiniumbetaine, deren Anwendungseigenschaften die der bekannten Produkte Übertreffen, sowie ein Verfahren zu ihrer Herstellung anzugeben, das die genannten Nachteile vermeidet und keine unsymmetrischen Diamine als Zwischenprodukte der Synthese erfordert. Dabei soll die Einführung der Sulfogruppe durch homogenkatalytische Hydrogensulfitaddition an Diallylammoniumsalze nach DD-A- 154 444 erfolgen und die Verwendung von Propansulton vermieden werden. Die Pyrrolidiniumsulfobetaine sollen als Tenside, Flotationsmittel sowie als Emulgatoren verwendbar sein. Die Aufgabe wird

anspruchsgemaß gelöst. Die abhängigen Ansprüche betreffen bevorzugte Ausführungsformen. Das erfindungsgemäße Verfahren zur Herstellung der Pyrrolidiniumsulfobetaine der allgemeinen Formel I ist gekennzeichnet durch

- Quaternisierung von Alkyldiallylaminen der allgemeinen Formel III

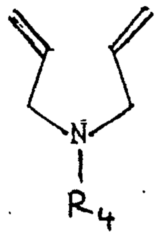

(III)

mit $R_4$ wie oben mit Halogencarbonsäureestern der allgemeinen Formel IV

$$Hal — CH(R_3) — (CH_2)_n — CO — O — Alkyl \qquad (IV)$$

mit $R_3$ und n wie oben und

- anschließende Umsetzung der entstandenen quartären Ammoniumverbindungen der Formel V

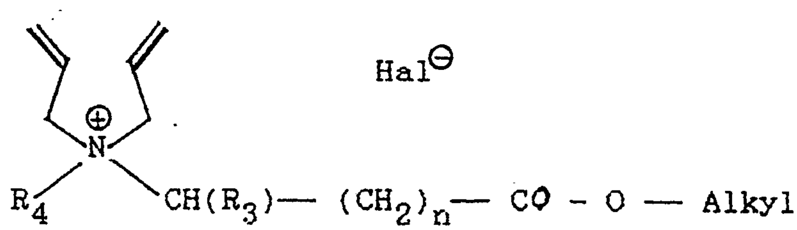

(V)

mit $R_3$, $R_4$ und n wie oben
mit Hydrogensulfit und mit Aminen der allgemeinen Formel VIII

$$HNR_1R_2 \qquad (VIII)$$

mit $R_1$ und $R_2$ wie oben in Gegenwart von Oxidationsmitteln, wobei die Reihenfolge der beiden letztgenannten Reaktionspartner verändert wer den kann.

Die Verfahrensschritte erfolgen entweder in der Reihenfolge, daß aus Alkyldiallylaminen der Formel III und Halogencarbonsäureestern der Formel IV quartäre Ammoniumsalze der Formel V erhalten werden, die mit entsprechenden Aminen der Formel VIII zu Amiden der Formel VI

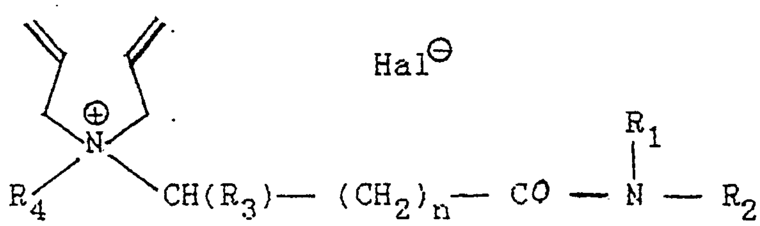

(VI)

reagieren, die dann durch Sulfocyclisierung, d. h. gleichzeitige Einwirkung von Hydrogensulfiten und Oxidationsmit teln, zu den Sulfobetainen der Formel I umgesetzt werden. Die Verfahrensschritte können

alternativ auch in der Reihenfolge durchgeführt werden, daß die Zwischenstufe der Formel V durch Sulfocyclisierung in die reaktionsfähige Zwischenstufe der Formel VII

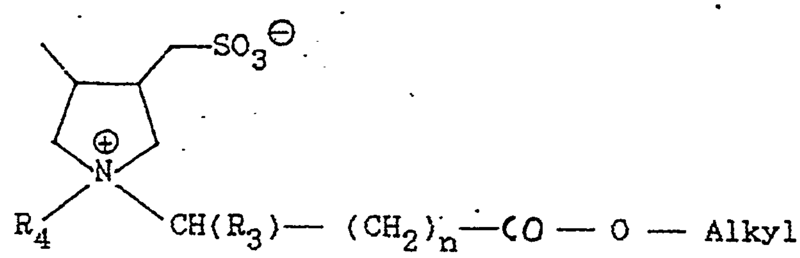

(VII),

übergeführt wird, die dann mit Aminen der Formel VIII zu den Zielverbindungen der Formel I umgesetzt wird. Die Zwischenstufe der Formel V kann in Einzelfällen auch so gewonnen werden, daß man N,N-Diallylaminocarbonsäuren alkyliert.

Die einzelnen Verfahrensschritte können ohne Nachteil auch ohne Isolierung der Zwischenstufen durchgeführt werden. Bevorzugte Lösungsmittel für die Umsetzung sind Wasser, Alkohole,Wasser-Alkohol-Gemische und in Einzelfällen auch andere polare Lösungsmittel. Bis zur Amidstufe VI kann auch ohne Lösungsmittel gearbeitet werden.

Die Verfahrensschritte werden vorzugsweise im Temperaturbereich von 10 bis 100 °C durchgeführt.

Die Bildung der quartären Ammoniumsalze erfordert in der Regel Temperaturen zwischen 20 und 100 °C, in den meisten Fällen reichen schon 50 °C für brauchbare Umsetzungsgeschwindigkeiten aus. Die Herstellung der Säureamidbindung erfolgt ebenfalls in diesem Temperaturbereich. Die Sulfocyclisierung zum Sulfobetain erfolgt durch Einwirkung von Hydrogensulfiten auf die Diallylammoniumsalze in Gegenwart von Oxidationsmitteln. Die Bildung additionsfähiger Sulfit anionradikale erfolgt vorzugsweise durch das System Luftsauerstoff/Übergangsmetallion. Sie gelingt jedoch auch in technisch brauchbarer Weise mit Salzen der Peroxodischwefelsäure, mit Alkyl- oder Acylperoxiden, Wasserstoffperoxid, Nitraten oder Nitriten als Oxidationsmittel.

Die neuen Pyrrolidiniumsulfobetaine der Formel I zeigen hervorragende Tensideigenschaften, wenn der Säureamidteil ein langkettiges Amin als Baustein enthält, was ihre Verwendung als Bestandteil in Wasch- und Reinigungsmitteln, als Flotationsmittel oder als Emulgatoren ermöglicht.

Die neuen Verbindungen der Formel I besitzen ein noch besseres Kalkseifendispergiervermögen als die bekannten Verbindungen der Formel II. Im Vergleich mit bekannten Sulfobetainen zeigen diese neuen Stoffe eine überraschend gute Löslichkeit in Kohlenwasserstoffen, Aromaten und Alkanolen, was die Anwendungsbreite dieses Tensidtyps erweitert.

Das erfindungsgemäße Verfahren ist in technischem Maßstab als Eintopfverfahren durchführbar, weil die bisher nicht technisch verfügbaren Aminkomponenten der Formel III jetzt nach DD-A-136 497 wohlfeil zur Verfügung stehen. Die nachfolgenden Syntheseschritte verlaufen überraschenderweise jeweils mit quantitativer Ausbeute; deshalb kann ohne Isolierung von Zwischenstufen gearbeitet werden, was gegenüber dem bekannten Stand der Technik einen wertvollen Verfahrensvorteil darstellt.

Das schwer zugängliche und hochgradig cancerogene Propansulton wird als Synthesekomponente nicht mehr benötigt. Gleichzeitig entfällt die Verwendung unsymmetrisch dialkylierter Diamine.

Im folgenden wird die Erfindung anhand von Ausführungsbeispielen näher erläutert.

## Beispiel 1

1,3-Dimethyl-l-dodecylaminocarbonylmethyl-4-sulfomethyl-pyrrolidinium-betain

N,N-Diallyl-N-methylammonioessigsäuremethylester-chlorid: 1112 g (10 mol) Methyldiallylamin werden in einem Rührgefäß vorgelegt. Dazu tropft man unter Rühren bei einer Starttemperatur von 35 °C 1085 g (10 mol) Chloressigsäuremethylester (frei von Chlorssigsäure). Während des Zutropfens steigt die Temperatur auf 70 °C an; die reagierende Mischung trübt sich und beginnt laufend das Quaternisierungprodukt als dickes, viskoses Öl auszuscheiden. Durch Regulierung der Zutropfgeschwindigkeit oder durch gelegentliches Kühlen wird die Reaktionstemperatur im Bereich von 70 bis 80 °C gehalten, so daß nach etwa 30 min der Chloressigsäuremethylester zugesetzt ist. Man läßt kurzzeitig in diesem Temperaturbereich nachreagieren, bis der pH-Wert des Reaktionsproduktes auf 7 abfällt. Es wird vollständiger Umsatz erzielt, da $^{1}$H-

NMR-spektroskopisch nur noch das Zielprodukt nachweisbar ist, welches als homogene, klar durchsichtige, sehr viskose Flüssigkeit anfällt.

N,N-Diallyl-N-methylammonioessigsäure-dodecylamid-chlorid:

Zu dem vorstehend beschriebenen Methylesterchlorid tropft man bei 70 °C 1853 g (10 mol) Dodecylamin (Cocosamin) unter Rühren und gelegentlichem Kühlen innerhalb von 30 min derart zu, daß die Reaktionstemperatur 80 °C nicht übersteigt. Das hochviskose Produkt zeigt dann einen pH-Wert von 7 und somit vollständigen Umsatz an.

3730 g des Dodecylamidchlorids werden mit 11,19 1 Leitungswasser zu einer 25%igen Lösung gelöst (Lösung I). Dann werden 950 g (5 mol) Natriummetabisulfit $Na_2S_2O_5$ und 126 g (1 mol) Natriumsulfit $Na_2SO_3$ in 14,07 1 Leitungswasser zu einer gepufferten Natriumhydrogensulfitlösung gelöst (Lösung II). In einem Rührgefäß mit Lufteinleitungsrohr werden 1 mol (126 g) Natriumsulfit in 5 1 Leitungswasser gelöst. Zu dieser Lösung tropft man nun unter Rühren und gleichzeitigem Einleiten von Luft bei 25 °C die Lösungen I und II simultan zu. Die bald eintretende Schaumbildung wird durch Zusatz von Isopropanol gemäßigt. Nach einer Zutropfzeit von ca. 90 min erhöht sich die Temperatur des Reaktionsgemisches auf 37 °C und fällt nach beendeter Sulfocyclisierung wieder ab. Danach wird unter weiterem intensiven Lufteinleiten und unter Rühren die Umsetzung vervollständigt. Die erhaltene Lösung des Pyrrolidiniumbetains kann jetztventweder sofort als Tensid eingesetzt oder durch Einengen und Abtrennen von Begleitsalzen mit Ethanol gereinigt werden. Das reine Produkt hat einen Schmelzpunkt von 162 - 165 °C (aus Ethanol).

Beispiel 2

1-Decylaminocarbonylmethyl-1,3-dimethyl-4-sulfomethyl-pyrrolidinium-betain

0,15 mol nach Beispiel 1 bereitetes N,N-Diallyl-N-methylammonioessigsäuremethylester-chlorid werden in analoger Arbeitsweise anstelle von Cocosamin mit 0,15 mol n-Decylamin umgesetzt und anschließend mit gepufferter Hydrogensulfitlösung und Luft sulfocyclisiert. Man erhält in quantitativer Ausbeute das entsprechende Pyrrolidiniumbetain vom Schmelzpunkt 163 - 168 °C.

Beispiel 3

1,3-Dimethyl-1-hexylaminocarbonylmethyl-4-sulfomethyl-pyrrolidinium-betain

0,1 mol des nach Beispiel 1 hergestellten H,H-Diallyl-N-methylammonioessigsäuremethylester-chlorids werden wie in Beispiel 1 und 2 beschrieben mit 0,1 mol n-Hexylamin als Aminkomponente zur Reaktion gebracht. Bei der Sulfocyclisierungsstufe ist eine Schaumminderung durch Isopropanolzusatz nicht erforderlich. Der Umsatz ist quantitativ. Schmelzpunkt >246 °C (unter Zersetzung).

Beispiel 4

1-Butylaminocarbonylmethyl-1,3-dimethyl-4-sulfomethyl-pyrrolidinium-betain

Man verfährt wie in den Beispielen 1 und 3 beschrieben unter Verwendung von n-Butylamin als Aminkomponente. Schmelzpunkt:> 263 °C (unter Zersetzung).

Beispiel 5

1,3-Dimethyl-1-isopropylaminocarbonylmethyl-4-sulfomethyl-pyrrolidinium-betain

$^{13}C$-NMR-Spektrum ($D_2O$, externer Standard TMS)

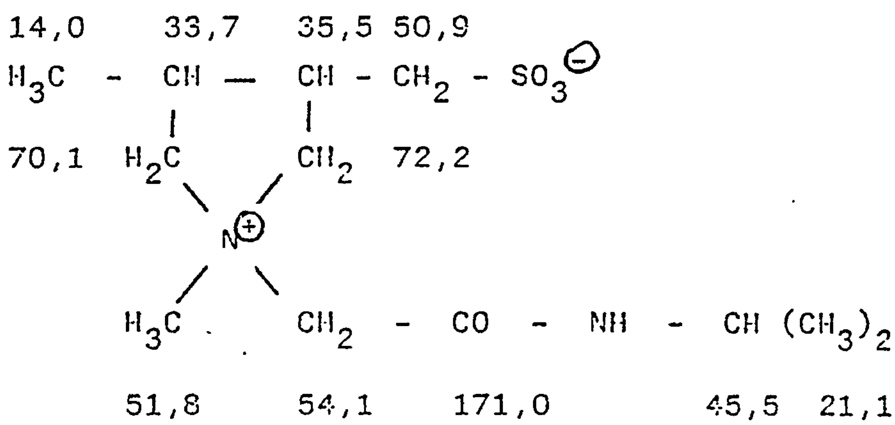

Die Zahlenangaben an den Atomsymbolen entsprechen den chemischen Verschiebungen in ppm: Hauptkomponente der cis-Strukturisomeren in Bezug auf die Substituenten in der 3,4-Position.
Man verfährt wie in Beispiel 1 und 3 beschrieben unter Verwendung von Isopropylamin als Aminkomponente. Der Umsatz ist quantitativ. Schmelzpunkt: 246° C (unter Zersetzung). Der Schmelzpunkt des entsprechenden n-Propylderivats beträgt >198° C (unter Zersetzung).

Beispiel 6

1,3-Dimethy-1-hydrazinocarbonylmethyl-4-sulfomethyl-pyrrolidinium-betain

Man verfährt wie in Beispiel 1 und 3 beschrieben unter Verwendung von 80%igem Hydrazinhydrat als Aminkomponente. Schmelzpunkt:>257° C (unter Zersetzung).

Beispiel 7

1-Aminocarbonylmethyl-1,3-dimethyl-4-sulfomethyl-pyrrolidinium-betain

Man verfährt wie in Beispiel 1 und 3 beschrieben, setzt jedoch anstelle des Chloressigsäuremethylesters und der Aminkomponente eine ethanolische Lösung von Chloracetamid ein. Die Sulfocyclisierung erfolgt anschließend wie in den vorstehenden Beispielen beschrieben. Schmelzpunkt:>274° C (unter Zersetzung).
$^{13}C$-NMR-Spektrum (Angaben wie in Beispiel 5):

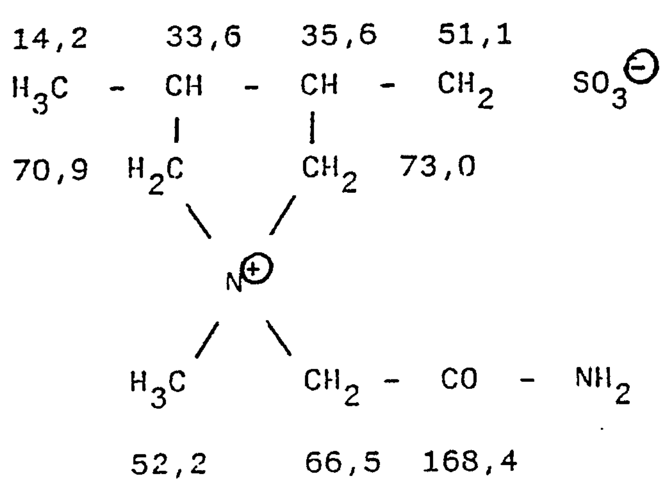

Beispiel 8

1,3-Dimethyl-1-hexadecylaminocarbonylmethyl-4-sulfomethyl-pyrrolidinium-betain

Man stellt, wie in Beispiel 1 beschrieben, 1 mol N,N-Diallyl-N-methylammonioessigsäuremethylester-chlorid her und versetzt dieses unter Rühren im Temperaturbereich 70-80° C portionsweise mit festem n-Hexadecylamin (1 mol) im Verlaufe von 10 min. Das erhaltene sirupöse Hexadecylamidchlorid wird bei 40° C in einem Isopropanol-Wasser-Gemisch gelöst und bei dieser Temperatur sulfocyclisiert. Die Ausbeute ist quantitativ. Das Produkt fällt nach dem Erkalten der Lösung als Feststoff aus. Schmelzpunkt: 128-137° C aus Ethanol. Bei der Herstellung des analogen Produktes aus $C_{16}/C_{18}$-Amin-Gemisch verfährt man ganz entsprechend. Schmelzpunkt dieses Pyrrolidiniumbetain-Gemisches: 86 - 94° C.

Beispiel 9

1-Bis(dodecylaminocarbonyl)-methyl-1,3-dimethyl-4-sulfomethylpyrrolidinium-betain

N,N-Diallyl-N-methylammoniomalonsäurediethylesterbromid:

111g (1 mol) Methyldiallylamin werden in 200 ml Ethanol gelöst; zu dieser Lösung tropft man unter Rühren bei 40° C 239 g (1 mol) Brommalonsäurediethylester und rührt 2 Stunden bei 80° C nach.

N,N-Diallyl-N-methylammoniomalonsäure-di-n-dodecylamidbromid:

Die wie vorstehend beschrieben erhaltene Ethylesterbromidlösung wird mit 370,6 g (2 mol) n-Dodecylamin (Cocosamin) umgesetzt, indem man das aufgeschmolzene Dodecylamin unter Rühren bei 80° C in die Lösung des Diethylesterbromids einträgt und zur Vervollständigung der Umsetzung noch 2 Stunden bei dieser Temperatur rührt. Die Sulfocyclisierung wird in der beschriebenen Arbeitsweise durchgeführt, wobei in Gegenwart von n-Butanol zur Verbesserung der Löslichkeit und bei 60° C gearbeitet wird. Nach Abkühlen des Reaktionsgemisches fällt das Pyrrolidiniumbetain als Feststoff aus und wird aus Ethanol umkristallisiert. Schmelzpunkt: 104 - 112° C.

Beispiel 10

Vergleichende Bestimmung des Kalkseifendispergiervermögens

Die Bestimmung erfolgt nach DDR-Standard TGL 22254 Blatt 1 (Gruppe 482) vom Juli 1977 (Determination of Lime-Soap Dispersing Property). Das Kalkseifendispergiervermögen ist die Fähigkeit, das Ausflocken oder Zusammenballen von Kalkseife zu verhindern und diese für eine bestimmte Zeit in so feiner Dispersion zu halten, daß sie sich nicht auf textilen oder anderen Oberflächen niederschlägt. Die Grenze der Beständigkeit von Seifenlösungen gegen hartes Wasser wird durch Zugabe verschiedener Mengen des zu prüfenden Produktes festgestellt. Der Berechnung wird die ermittelte Menge des geprüften Produktes zugrundegelegt. Die Angabe des Kalkseifendispergiervermögens K erfolgt in %, bezogen auf das als Standardbezugssubstanz eingesetzte Natriumoleat:

$$K = \frac{\text{Menge des eingesetzten Produktes in g}}{\text{Menge des eingesetzten Natriumoleats in g}} \times 100\ (\%)$$

d.h. niedrige Prozentwerte weisen ein gegenüber NatriumOleat gutes Kalkseifendispergiervermögen aus. Die Bestimmung erfolgte bei einer Wasserhärte von 30° dH.

| | K (%) |
|---|---|
| Natriumoleat (Standardbezugssubstanz) | 100 |
| 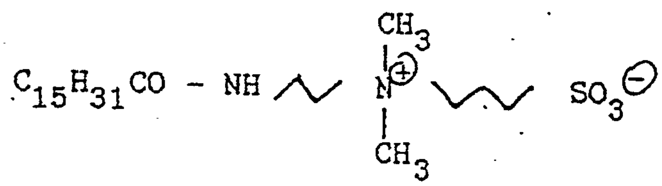 | |
| (Vergleichssubstanz nach Linfield et al., J. Amer. Oil. Chem. Soc. 55(1978) 87, 741) | 55,2 |
| 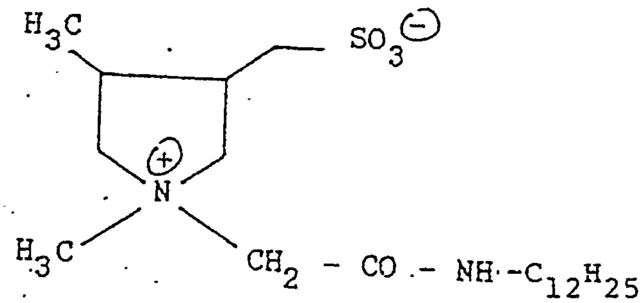 | |
| hergestellt nach Beispiel 1 | 35,2 |
| 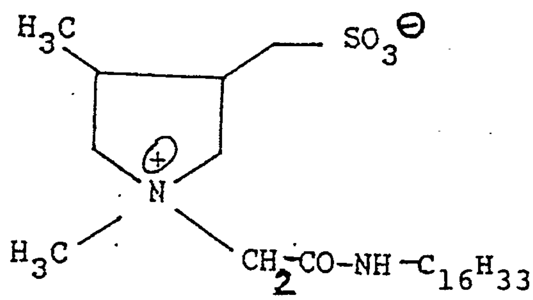 | |
| hergestellt nach Beispiel 8 | 5,2 |

**Ansprüche**

1. Pyrrolidiniumsulfobetaine mit Carbonamidgruppen der allgemeinen Formel I,

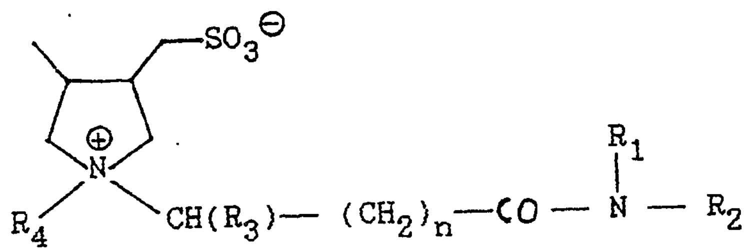

(I),

worin bedeuten:

- $R_1$ Wasserstoff, geradkettiges oder verzweigtes Alkyl mit bis zu 22 C-Atomen,
- $R_2$ Wasserstoff, $-NH_2$ oder Alkyl mit bis zu 4 C-Atomen,
- $R_3$ Wasserstoff, Alkylaminocarbonyl mit bis zu 22 C-Atomen oder Alkyl mit bis zu 22 C-Atomen,
- $R_4$ Wasserstoff oder Alkyl oder Hydroxyalkyl mit bis zu 22 C-Atomen und
- n 0, 1, 2 oder 3.

**2.** Verfahren zur Herstellung der Pyrrolidiniumsulfobetaine mit Carbcnamidgruppen nach Anspruch 1, gekennzeichnet durch

- Quaternisierung von Alkyldiallylaminen der allgemeinen Formel III,

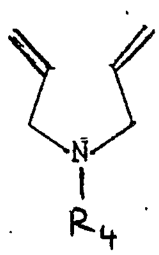

(III),

worin $R_4$ Wasserstoff oder Alkyl oder Hydroxyalkyl mit bis zu 22 C-Atomen bedeutet, mit Halogencarbonsäureestern der allgemeinen Formel IV,

$$Hal - CH(R_3) - (CH_2)_n - CO{-}O - Alkyl \qquad (IV),$$

worin $R_3$ Wasserstoff, Alkylaminocarbonyl mit bis zu 22 C-Atomen oder Alkyl mit bis zu 22 C-Atomen bedeutet,

und

- anschließende Umsetzung der entstandenen quartären Ammoniumverbindungen der Formel V,

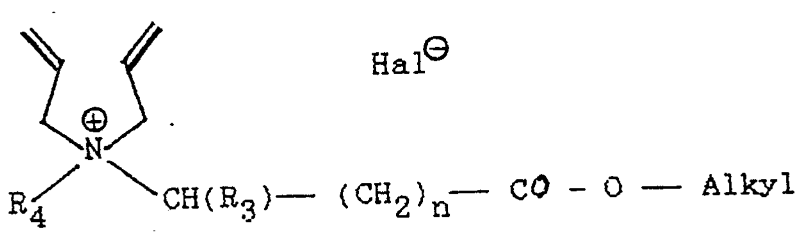

(V)

worin $R_3$ und $R_4$ dasselbe wie oben und n 0, 1, 2 oder 3 bedeuten, mit Hydrogensulfit und mit Aminen der allgemeinen Formel VIII,

$$HNR_1R_2 \qquad (VIII),$$

worin $R_1$ Wasserstoff, geradkettiges oder verzweigtes Alkyl mit bis zu 22 C-Atomen und

$R_2$ Wasserstoff, $-NH_2$ oder Alkyl mit bis zu 4 C-Atomen bedeuten,
in Gegenwart von Oxidationsmitteln.

3. Verfahren nach Anspruch 2, dadurch gekennzeichnet, daß die Verfahrensschritte im Temperaturbereich von 10 bis 100 °C durchgeführt werden.

4. Verfahren nach Anspruch 2 oder 3, dadurch gekennzeichnet, daß die Reaktionen in polaren Medien durchgeführt werden.

5. Verfahren nach einem der Ansprüche 2 bis 4, dadurch gekennzeichnet, daß die quartären Ammoniumverbindungen zunächst mit Hydrogensulfit der Sulfocyclisierung unterworfen und anschließend mit Aminen der Formel VIII umgesetzt werden.

6. Verfahren nach einem der Ansprüche 2 bis 4, dadurch gekennzeichnet, daß die quartären Ammoniumverbindungen zunächst mit Aminen der Formel VIII umgesetzt und anschließend mit Hydrogensulfit der Sulfocyclisierung unterworfen werden.

7. Verfahren nach einem der Ansprüche 2 bis 6, dadurch gekennzeichnet, daß für die Sulfocyclisierung als Oxidationsmittel Luftsauerstoff in Gegenwart von Übergangsmetallionen, Peroxodisulfat, Alkyl- oder Acylperoxide, Wasserstoffperoxid, Nitrate oder Nitrite verwendet werden.

8. Verwendung der Pyrrolidiniumsulfobetaine der Formel I nach Anspruch 1 als Tenside in Wasch- und Reinigungsmitteln, als Flotationsmittel sowie als Emulgatoren.

## Claims

1. Pyrrolidinium sulphobetaines containing carboxamide groups, of the general formula I

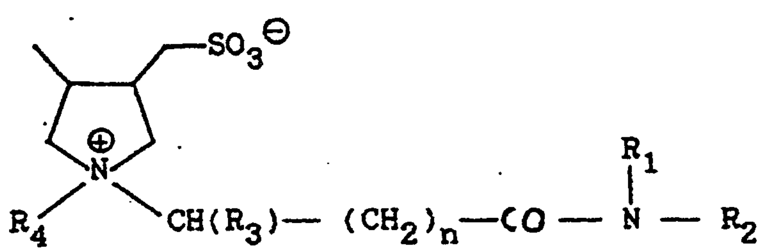

(I)

wherein:

$R_1$ denotes hydrogen or straight-chain or branched alkyl having up to 22 C atoms,
$R_2$ denotes hydrogen, $-NH_2$ or alkyl having up to 4 C atoms,
$R_3$ denotes hydrogen, alkylaminocarbonyl having up to 22 C atoms or alkyl having up to 22 C atoms,
$R_4$ denotes hydrogen or alkyl or hydroxyalkyl having up to 22 C atoms and
n denotes 0, 1, 2 or 3.

2. Process for the preparation of the pyrrolidinium sulphobetaines containing carboxamide groups according to claim 1,
characterized in that
- alkyldiallylamines of the general formula III

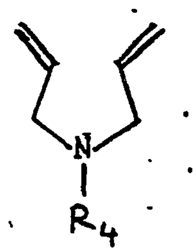

(III)

wherein $R_4$ denotes hydrogen or alkyl or hydroxyalkyl having up to 22 C atoms, are quaternized with halogenocarboxylic acid esters of the general formula IV

$$Hal - CH(R_3) - (CH_2)_n - CO - O - Alkyl \qquad (IV)$$

wherein $R_3$ denotes hydrogen, alkylaminocarbonyl having up to 22 C atoms or alkyl having up to 22 C atoms,

and

- the quaternary ammonium compounds formed, of the formula V

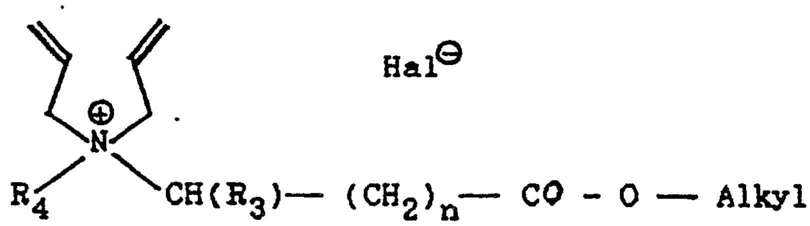

(V)

wherein $R_3$ and $R_4$ have the same meanings as above and n denotes 0, 1, 2 or 3 are then reacted with hydrogen sulphite and with amines of the general formula VIII

$$HNR_1R_2 \qquad (VIII)$$

wherein $R_1$ denotes hydrogen or straight-chain or branched alkyl having up to 22 C atoms and $R_2$ denotes hydrogen, $-NH_2$ or alkyl having up to 4 C atoms in the presence of oxidizing agents.

3. Process according to claim 2, characterized in that the process steps are carried out in the temperature range from 10 to 100° C.

4. Process according to claim 2 or 3, characterized in that the reactions are carried out in polar media.

5. Process according to one of claims 2 to 4, characterized in that the quaternary ammonium compounds are first subjected to the sulphocyclization with hydrogen sulphite and the products are then reacted with amines of the formula VIII.

6. Process according to one of claims 2 to 4, characterized in that the quaternary ammonium compounds are first reacted with amines of the formula VIII and the products are then subjected to the sulphocyclization with hydrogen sulphite.

7. Process according to one of claims 2 to 6, characterized in that atmospheric oxygen in the presence of transition metal ions, peroxodisulphate, alkyl or acyl peroxides, hydrogen peroxide, nitrates or nitrites are used as oxidizing agents for the sulphocyclization.

8. Use of the pyrrolidinium sulphobetaines of the formula I according to claim 1 as surfactants in detergents and cleaning agents, as flotation agents and as emulsifiers.

## Revendications

**1.** Pyrrolidiniumsulfobétaïnes comportant un groupe carbonamido de formule générale I

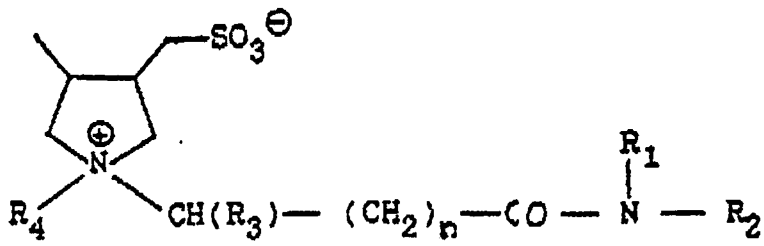

(I),

dans laquelle:

- $R_1$ désigne un atome d'hydrogène ou un groupe alkyle à chaîne droite ou ramifiée comportant au maximum 22 atomes de carbone;
- $R_2$ désigne un atome d'hydrogène, un groupe $-NH_2$ ou alkyle comportant 4 atomes de carbone au maximum;
- $R_3$ désigne un atome d'hydrogène, un groupe alkylaminocarbonyle comportant au plus 22 atomes de carbone ou alkyle comportant au plus 22 atomes de carbone;
- $R_4$ désigne un atome d'hydrogène ou un groupe alkyle ou hydroxyalkyle comportant au plus 22 atomes de carbone et
- n équivaut à 0, 1, 2 ou 3.

**2.** Procédé de préparation des pyrrolidiniumsulfobétaïnes comportant un groupe carbonamido selon la revendication 1, caractérisé par les opérations de:

- quaternisation d'alkyldiallylamines de formule générale III

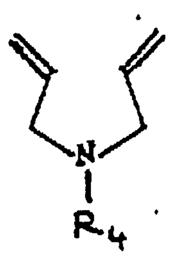

(III),

où $R_4$ désigne un atome d'hydrogène ou un groupe alkyle ou hydroxyalkyle comportant au maximum 22 atomes de carbone,

avec des esters d'acides carboxyliques halogénes de formule générale IV,

$$\text{Hal} - CH(R_3) - (CH_2)_n - CO - O - \text{Alkyl} \qquad (IV),$$

où $R_3$ désigne un atome d'hydrogène, un groupe alkylaminocarbonyle comportant au maximum 22 atomes de carbones ou alkyle comportant au maximum 22 atomes de carbones, et

- réaction subséquente des composés d'ammonium quaternaires de formule V, ainsi formés

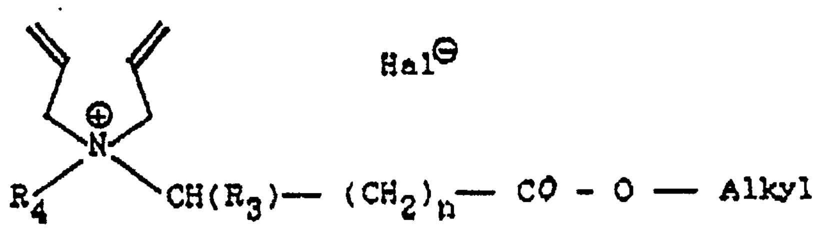

(V)

où $R_3$ et $R_4$ ont les significations données précédemment et n équivaut à 0, 1, 2 ou 3, avec de l'hydrogénosulfite et des amines de formule générale VIII,

$$HNR_1R \qquad (VIII),$$

où $R_1$ désigne un atome d'hydrogène ou un groupe alkyle à chaîne droite ou ramifiée comportant au maximum 22 atomes de carbone et $R_2$ désigne un atome d'hydrogène, un groupe $NH_2$ ou alkyle comportant au maximum 4 atomes de carbone,

en présence d'agents d'oxydation.

**3.** Procédé selon la revendication 2, caractérisé en ce qu'on effectue les opérations dans une plage de températures de 10 à 100 ° C.

**4.** Procédé selon la revendication 3 ou 3, caractérisé en ce qu'on effectue les réactions en milieu polaire.

**5.** Procédé salon l'une des revendications 2 á 4, caractérisé en ce que les composés d'ammonium quaternaires sont d'abord soumis à la sulfocyclisation à l'aide d'hydrogénosulfite puis mis en réaction avec des amines de formule VIII.

**6.** Procédé selon l'une des revendications 2 à 4, caractérisé en ce que les composés d'ammonium quaternaires sont d'abord mis en réaction avec les amines de formule VIII puis soumis à la sulfocyclisation à l'aide d'hydrogénosulfite.

**7.** Procédé selon l'une des revendications 2 à 6, caractérisé en ce qu'on utilise pour la sulfocyclisation, en tant qu'oxydant, l'oxygène de l'air en présence d'ions des métaux de transition, un peroxodisulfate, des alkyl- ou acylperoxydes, l'eau oxygénée, des nitrates ou des nitrites.

**8.** Utilisation des pyrrolidiniumsulfobétaïnes de formule 1 selon la revendication 1 comme tensides dans des agents de lavage et de nettoyage, comme agents de flottation ainsi que comme émulsifiants.